# EUROPÄISCHE PATENTSCHRIFT

(11) **EP 3 185 891 B1**
(45) Veröffentlichungstag und Bekanntmachung des Hinweises auf die Patenterteilung: **08.04.2020**
(21) Anmeldenummer: 15753942.0
(22) Anmeldetag: 20.08.2015
(51) Int. Cl.: A61K 38/48, C12N 9/50, C12N 9/64, A61P 41/00

(54) **REKOMBINANTE FUSIONSPROTEINE ZUR VORBEUGUNG ODER BEHANDLUNG VON ADHÄSIONEN BEI GEWEBEN ODER ORGANEN**
RECOMBINANT FUSION PROTEINS FOR PREVENTING OR TREATING ADHESIONS OF TISSUES OR ORGANS
PROTÉINES DE FUSION RECOMBINANTES POUR LA PRÉVENTION OU LE TRAITEMENT DES ADHÉRENCES DES TISSUS OU DES ORGANES

(30) Priorität: 26.08.2014 DE 102014112212
(43) Veröffentlichungstag der Anmeldung: 05.07.2017
(73) Patentinhaber: Akesion GmbH, 69198 Schriesheim (DE)
(72) Erfinder: RÜBSAMEN, Klaus, 67434 Neustadt (DE); WITTE, Stephan, 69198 Schriesheim (DE)
(74) Vertreter: Patentanwälte Dr. Keller, Schwertfeger Partnerschaft mbB
(86) Internationale Anmeldenummer: PCT/EP2015/069176
(87) Internationale Veröffentlichungsnummer: WO 2016/030278

(56) Entgegenhaltungen:
- EP-A1- 0 395 375
- WO-A1-95/15747
- WO-A1-99/29838
- US-B1- 6 461 640
- XIUPING JIANG ET AL: "Soluble expression, purification, and characterization of Gloydius shedaoensis venom gloshedobin in Escherichia coli by using fusion partners", APPLIED MICROBIOLOGY AND BIOTECHNOLOGY, SPRINGER, BERLIN, DE, Bd. 85, Nr. 3, 29. Juli 2009 (2009-07-29), Seiten 635-642, XP019778503, ISSN: 1432-0614

## Beschreibung

Unter normalen Heilungsbedingungen reagiert der Säugetierorganismus auf eine Verletzung mit spezifisch ablaufenden Wundheilungsprozessen. Diese führen zur Bildung von Fibrin, einer klebrigen, für den Wundverschluss verantwortlichen Substanz. Fibrinogen, die Vorstufe des Fibrins, zirkuliert im Blut und tritt im Bereich der Verletzung als Wundflüssigkeit (Exsudat) aus. Durch die Einwirkung von lokal gebildetem Thrombin wird Fibrinogen in unlösliches Fibrin umgewandelt, so dass sich die Wunde innerhalb wenigen Minuten verschließt. Überschüssiges Fibrin wird durch das gleichzeitig aktivierte endogene fibrinolytische System durch die Wirkung von Plasmin zügig abgebaut.

### Technisches Gebiet:

Gewebeverklebungen oder Adhäsionen sind das Ergebnis eines fehlgeleiteten Wundheilungsprozesses, das auf ein Ungleichgewicht des endogenen fibrinolytischen Systems zurückzuführen ist (Hellebrekers et al., 2005; Hellebrekers & Kooistra, 2011). Bei unfall- oder operativbedingten Verletzungen im Bauchraum ist der Abbau von Fibrin durch die Aktivierung von Inhibitoren von Plasmin, insbesondere durch PAI-1, PAI-2 und α₂-Antiplasmin, beeinträchtigt. Überschüssiges, nicht abgebautes Fibrin führt in Folge zu Verklebungen benachbarter Gewebe und Organe, die sich innerhalb weniger Tage durch das Einwachsen von Fibroblasten und Blutgefäßen in dauerhafte Verwachsungen umwandeln (Arung et al., 2011). Diese Verwachsungen können sowohl zwischen einzelnen Organen als auch zwischen Organen und dem Peritoneum entstehen. Die dadurch eingeschränkte Verschiebbarkeit der Organe macht sich in vielen Fällen durch chronische Schmerzen oder, bei weiblichen Patienten, durch Unfruchtbarkeit bemerkbar und kann in schweren Fällen durch die Beeinträchtigung der Darmperistaltik zu einem lebensbedrohlichen Darmverschluss führen.

### Stand der Technik:

Eine retrospektive Analyse der Scottish National Health Service Medical Record Linkage Database ergab, dass bei weit über der Hälfte aller operativen Eingriffe im Bauchraum Verwachsungen auftreten (Ellis et al. 1999). Bei etwa einem Drittel der Patienten mit Verwachsungen waren die klinischen Symptome so schwerwiegend, dass diese bereits im ersten Jahr nach der Operation erneut operiert werden mussten. Die hohe Zahl der Mehrfachoperationen zeigt, dass das Risiko weiterer Adhäsionen mit der Anzahl der Operationen signifikant zunimmt (Parker et al, 2001, 2005).

Eine Metaanalyse von 196 Studien mit insgesamt 150.797 Patienten ergab, dass nach 9 % der Bauchoperationen ein Darmverschluss als schwerwiegende Komplikation auftrat (Broek et al., 2013a), wobei die adhäsionsbedingte Ursache bei 2 % der Darmverschlüsse intraoperativ bei Notoperation bestätigt wurde. Bei 6 % der Nachoperationen zur Entfernung von Verklebungen wurden schwerwiegende Folgeschäden an inneren Organen verursacht. Verletzungen des Darms bei der chirurgischen Entfernung von Adhäsionen stellten dabei den wichtigsten isolierten Risikofaktor dar (Brummer et al. 2011). Die zusätzlich auftretenden Kosten des durch Verklebungen verursachte Zeit- und Materialaufwands sind beträchtlich. Bei 138 Einweisungen aufgrund von postoperativen intestinalen Obstruktionen (Kössi et al. 2003) waren insgesamt 1118 stationäre Behandlungstage erforderlich.

Auf der Basis von Daten der "2005 Healthcare Cost and Utilization Project's Nationwide Inpatient Sample" berechneten Sikirica et al. (2011) Gesamtkosten von 2,3 Mrd. USD für die Behebung adhäsionsbedingter Operationsfolgen. Davon entfielen 1,4 Mrd. USD auf die primäre Adhäsiolyse und 926 Mio. USD auf sekundäre Adhäsiolysen. Die adhäsionsbedingten Folgen von Bauchoperationen verursachten insgesamt 57.005 zusätzliche Krankenhaustage. Eine Analyse der Krankheitsdaten auf der Basis der US "National Hospital Discharge Database", 1994, ergab, dass zur nachträglichen Entfernung von Verwachsungen insgesamt 846.415 zusätzliche Krankenhaustage erforderlich waren, die zusätzliche Kosten von 1,3 Mrd. USD pro Jahr verursachten (Ray et al., 1998).

Diese pharmakoökonomischen Daten belegen die Notwendigkeit und den potentiellen Nutzen einer effektiven und sicheren Adhäsionsprophylaxe sowohl für den einzelnen Patienten als auch für das Gesundheitssystem. Die einzig mögliche Therapie von peritonealen Verwachsungen besteht in der chirurgischen Trennung der verwachsenen Oberflächen und Organe. Aufgrund des durch den erneuten Eingriff erhöhten Risikos für die Entstehung weiterer Verwachsungen kommt prophylaktischen Methoden, die geeignet sind, das Risiko für die Entstehung von Verklebungen zu verringern, besondere Bedeutung zu.

Als prophylaktische Methoden zur Verhinderung von Verklebungen und Verwachsungen kann entweder die Operationstechnik optimiert oder können mechanische Methoden zur physikalischen Trennung der verletzten Oberflächen eingesetzt werden. Schließlich gibt es pharmakologische Ansätze zur Beeinflussung der zugrundeliegenden pathophysiologischen Prozesse (zusammengefasst in Arung et al., (2011); Schnüriger et al., (2011)).

Durch den Einsatz minimal invasiver Operationstechniken (Laparoskopie) scheint das Risiko von Verklebungen und Verwachsungen im Vergleich zu der konventionellen Operationstechnik mit geöffnetem Bauchraum (Laparotomie) etwas geringer zu sein (Broek et al., 2013b; Schnüriger er al., 2011). Der Unterschied ist vermutlich bedingt durch die im Vergleich zur Laparotomie kleinere Wundfläche, geringere Manipulation der Gewebe und Organe, sowie das generell geringere Infektionsrisiko dieser Operationstechnik (SCAR Group, 2013). Trotz der zahlreichen Verbesserungen der chirurgischen Technik stellen Verklebungen als Folge operativer Eingriffe im Bauchraum ein unverändert hohes Risiko für Komplikationen wie z. B. chronische Schmerzen, Unfruchtbarkeit oder sogar Darmverschluss dar (Wallwiener et al., 2014).

Eine weitere derzeit verwendete Methode zur Verringerung des Risikos postoperativer Adhäsionen ist die mechanische Trennung der verletzten Gewebebereiche durch Einsatz von Barrieren aus unterschiedlichen biokompatiblen Materialien oder durch die Instillation einer größeren Flüssigkeitsmenge im Bauchraum. Die am häufigsten verwendete Maßnahme ist das Einbringen dünner Membranen aus bioabbaubaren Materialien, die zwischen die verletzten Oberflächen eingebracht und dort fixiert werden. Durch die mechanische Trennung der Organoberflächen können diese isoliert abheilen, was zur Prävention von Verklebungen führen soll. Zusammensetzungen wie Hyaluronsäure- und Carboxymethylzellulose (Seprafilm) werden beispielsweise als Membran auf die verletzte Oberfläche aufgelegt. Ferner wurde Hyaluronsäure zur Verhinderung von Adhäsionen bei Myomektomie eingesetzt, was zu einer Reduktion der Verklebungen führte (Fossum et al. 2011). Weitere bekannte Zusammensetzungen sind gelbildende Polyethylenglykole, Polyethylenoxid in Kombination mit Natriumcarboxymethylzellulose oder oxygenierte regenerierte Zellulose. Weitere Beispiele für derartige Adhäsionsbarrieren sind in der Offenlegungsschrift US 8629314A, sowie in Form einer neuartigen mehrschichtigen Adhäsionsbarriere in der US 20080254091A beschrieben.

Einige experimentelle Ansätze befassen sich mit der Verwendung von chemischen Substanzen mit dem Ziel, in den pathophysiologischen Prozess der Adhäsionsbildung in einem frühen Stadium einzugreifen. Diese initiale, nur wenige Stunden dauernde Phase der Adhäsion ist gekennzeichnet durch eine anhaltende lokale Entzündung und Gerinnungsaktivierung, gefolgt von erhöhter Gefäßpermeabilität, die zur Ansammlung eines fibrinreichen, die Oberfläche bedeckenden Exsudats führt (Hellebrekers und Kooistra 2011). Zustände wie Gewebehypoxie (Saed und Diamond 2004) und die durch Makrophagen, T-Zellen und die durch Cytokine induzierte zelluläre Immunreaktion sind verstärkende Faktoren für die Bildung von Adhäsionen (Binnebösel et al. 2011). Bereits am zweiten Tag ist die Wundoberfläche von Makrophagen, Fibroblasten und Mesothelzellen bedeckt, die sich in kurzer Zeit zu einer geschlossenen Schicht vereinen, durchsetzt von Mastzellen, Fibroblasten und neu gebildeten Endothelzellen. Diese irreversible Bildung einer festen Matrix ist die Grundlage des weiteren Zusammenwachsens, charakterisiert durch bandförmige Kollagenfibrillen und das Einwachsen von Blutgefäßen und Bindegewebsfasern.

Das zentrale Ereignis bei der Bildung von Adhäsionen ist somit die Bildung einer lokalen Fibrinschicht aus der Vorstufe Fibrinogen. Unter normalen Bedingungen besteht ein Gleichgewicht zwischen Fibrinbildung und Fibrinabbau durch Plasmin, dessen enzymatische Aktivität wiederum durch die Inhibitoren PAI-1, PAI-2 und α₂-Antiplasmin geregelt wird. Experimentelle Daten zeigen, dass Fibroblasten, die im Bereich von Adhäsionen auftreten, eine verringerte Konzentration von Plasminogen Aktivator bei erhöhter Plasminogen Aktivator-Inhibitor-Bildung aufweist, was die unkontrollierte Entstehung einer Fibrin-Matrix begünstigt (Diamond et al. 2004). Diese Beobachtung wurde in einer prospektiven Studie an Patienten mit Endometriose bestätigt (Hellebrekers et al. 2005).

Die Hemmung der Entzündungsreaktion, die Erhöhung der fibrinolytischen Aktivität und die Beeinflussung der Gerinnung schienen somit geeignete therapeutische Angriffspunkte zu sein, um die Entstehung von Adhäsionen zu verhindern. In zahlreichen Experimenten wurde daher versucht, durch Verabreichung fibrinolytisch wirksamer Substanzen den Mangel an endogener fibrinolytischer Aktivität zu kompensieren. Die EP 0318801B1, US 5578305A, EP 0297860B1 und EP 0227400B1 beschreiben beispielsweise die Verwendung von rt-PA in Hydroxyethylzellulose Hydrogel und anderen Matrizes als wirksames Mittel zur Verhinderung von Adhäsionen. Auch r-tPA-Modifikationen mit erhöhter Fibrinaffinität wurden für diese Anwendung vorgeschlagen (EP 0517756B1). Die in der EP 0874634B1 beschriebene Hemmung der Fibrinbildung durch Verwendung von Thrombininhibitoren stellt einen weiteren Ansatz dar, die Adhäsion in frühem Stadium zu verhindern. Die EP 0473689B1 beschreibt die Verwendung von Plasminogenaktivatoren wie Urokinase, Streptokinase und t-PA und verweist auf die geringe Halbwertszeit im Blut. Als Lösung wird die Kopplung von Plasminogenaktivatoren an ein Fibrinfragment vorgeschlagen.

Durch wiederholte intraperitoneale Applikation von rt-PA konnte die Bildung von Adhäsionen in einem Mäusemodell verringert werden, wobei die Wirkung allerdings nicht dosisabhängig war (Binda et al. 2009). In einem Adhäsionsmodell an Ratten wurde der Plasminogen-Aktivator t-PA, Urokinase oder Streptokinase über 4 Tage entweder mit Hilfe einer biodegradierbaren Hydrogel Matrix oder durch 4 x tägliche i.p. Injektionen verabreicht. Das aus einer Matrix freigesetzte t-PA reduzierte die Adhäsionen von 72 ± 15 % (Kontrolle) auf 4 ± 3 % während intraperitoneale Injektionen Adhäsionen lediglich auf 49 ± 8 % reduzierten (Hill-West et al. 1995). Zusammengefasst sind die mit fibrinolytischen Substanzen erzielten Ergebnisse unbefriedigend.

Trotz dieser negativen experimentellen Befunde wurde rt-PA in einer prospektiven Studie an 26 Patienten mit Myomektomie unter klinischen Bedingungen getestet (Hellebrekers et al. 2009). Ein signifikanter Unterschied in der Häufigkeit von Adhäsionen zwischen den beiden Gruppen konnte nicht beobachtet werden. Die Häufigkeit und der Schweregrad der Adhäsionen waren mit den vor der Operation gemessenen PAI-1-Konzentrationen korreliert und weisen damit auf die Bedeutung des fibrinolytischen Systems für die Adhäsionsbildung hin. Für eine erfolgreiche Prävention sind jedoch relativ hohe t-PA- Konzentrationen erforderlich, die in dieser Studie aufgrund der kurzen biologischen Halbwertszeit von rt-PA nicht erreicht wurden. Somit sind auch diese Substanzen für einen präventiven oder therapeutischen Einsatz zur Vermeidung von Verklebungen in Gewebekompartimenten nach Verletzungen der Bauchhöhle nicht geeignet.

Alle bisher verwendeten pharmakologischen Methoden zur Verringerung des Risikos von Verklebungen erwiesen sich somit in klinischen Studien generell als wenig wirksam. Insbesondere die Wirksamkeit von fibrinolytisch wirksamen Substanzen war enttäuschend. Dies ist vermutlich darauf zurückzuführen, dass die fibrinolytische Aktivität exogen zugeführter fibrinolytischer Substanzen durch die bei Verklebungen auftretenden hohen Konzentrationen von Plasminogen-Aktivator-Inhibitor (PAI-1, PAI-2) gehemmt wird. Um eine ausreichende Wirksamkeit fibrinolytischer Substanzen zu erzielen, sind sehr hohe Dosierungen erforderlich, die aber aufgrund der Resorption dieser Substanzen durch die Peritonealmembran zu systemischen Nebenwirkungen, insbesondere Blutungen, führt. Die nebenwirkungsfreie Verhinderung bzw. die frühzeitige Auflösung der entstehenden Fibrin-Brücken als Ursache von Verklebungen ist damit nicht gewährleistet.

Aussichtsreicher erscheint somit die Entfernung von Fibrinogen, der Vorstufe von Fibrin, um dessen Bildung bereits im Anfangsstadium zu hemmen. Die Eignung von fibrinogenolytischen Enzymen zur Prophylaxe postoperativer Adhäsionen wurde beispielhaft mit dem aus dem Gift der Malayischen Grubenotter, *Calloselasma rhodostoma,* isolierten Enzym Ancrod tierexperimentell untersucht (Ashby et al, 1969; Buckman etal., 1975; Chowdhury, & Hubbell, 1996). Ancrod spaltet spezifisch die Arginin-Glycin-Bindung der Aα-Kette von Fibrinogen. Im Unterschied zu Thrombin wird die Bβ-Kette durch Ancrod nicht gespalten. Die daraus resultierenden des-A-Fibrinmonomere polymerisieren nach Abspaltung der Fibrinopeptide zu kurzkettigem, löslichem Fibrin, das nicht quervernetzt ist und durch Plasmin und das retikuloendotheliale System schnell eliminiert wird. Durch intravenöse oder intraperitoneale Applikation von Ancrod kann somit die Fibrinogenkonzentration im Blut kontrolliert herabgesetzt werden. Dies führte in den zitierten Untersuchungen zu einer nahezu vollständigen, dosisabhängigen Hemmung der Adhäsionsbildung. Aufgrund des geringen Molekulargewichtes von < 40 kDa kann Ancrod schnell aus der Bauchhöhle in das Blutkreislaufsystem übertreten und führt dort zur Hemmung der Gerinnung. Bei entsprechend hoher Blutkonzentration kann dies zu lokalen Blutungen führen.

Daneben sind auch pharmazeutische Zusammensetzungen eines Fusionsproteins von Ancrod bekannt, welche zur Auflösung von Blutkoagulaten eingesetzt werden. So beschreibt die EP 0 395 375 A1 ein Fusionsprotein zwischen Ancrod und einem 5'-terminalen Polypeptid, welches 2 bis 1000 Aminosäuren umfassen kann. Eine Verwendung dieses Fusionsproteins zur Behandlung postoperativer Adhäsionen ist darin jedoch nicht beschrieben.

In gleicher Weise wirkt das aus dem Gift der südamerikanischen Schlange *Bothrops atrox* gewonnene Batroxobin, welches ebenfalls selektiv Fibrinogen spaltet und dadurch die Fibrinogenkonzentration verringert. Ein Fusionsprotein zwischen Batroxobin und einer zweiten Polypeptidkette ist beispielsweise in der WO 99/29838 A1 beschrieben.

Ancrod wurde viele Jahre unter anderem zur Thromboseprophylaxe bei Patienten mit heparininduzierter Thrombozytopenie und zur Behandlung der peripheren arteriellen Durchblutungsstörung eingesetzt, inzwischen jedoch durch neuere Medikamente ersetzt. Diese und vergleichbare Enzyme sind jedoch trotz ihrer guten Wirksamkeit im Tiermodell zur Prophylaxe peritonealer Adhäsionen ungeeignet, da diese aufgrund ihrer Molekülstruktur nicht ausreichend lange in der Bauchhöhle verbleiben und schnell in den Blutkreislauf gelangen und die Gerinnung hemmen. Diese Nebenwirkung ist für die Indikation "Prophylaxe postoperativer Adhäsionen" prohibitiv, so dass eine native Anwendung dieser Substanzen bei Patienten nicht in Betracht kommt.

Daneben gibt es weitere fibrinogenolytische Fusionsproteine, die jedoch nicht mit dem Ansatz einer Behandlung peritonealer Adhäsionen entwickelt wurden. Solche Konstrukte sind beispielsweise in der US 6,214,594 B1 oder in Yang et al., Protein Expression and Purification, 66 (2009): 138-147 beschrieben.

Um eine langsame Freisetzung der Wirkstoffe und eine längere Wirkdauer im Peritonealraum zu erreichen, wurden beispielsweise fibrinolytische und defibrinogenierende Substanzen in einem Trägermaterial eingebettet (US 2004/224006 A1). Die US 8,629,314 B2 beschreibt die Einbettung verschiedener Wirksubstanzen in eine Polymermatrix zur Verhinderung von Adhäsionen. Ähnliche Versuche sind auch in der US 6,461,640 B1 und der WO 1995/15747 A1 offenbart. Hierbei wird eine topisch applizierte bioabbaubare Polymermatrix als Träger für Plasminogen, Urokinase und Streptokinase sowie Ancrod eingesetzt. Der Erfolg dieser Maßnahmen war gering, vermutlich hauptsächlich aufgrund der unkontrollierten Freisetzung der eingebetteten Wirkstoffe.

### Darstellung der Erfindung:

Vor diesem Hintergrund ist es Aufgabe der vorliegenden Erfindung, die bei bekannten fibrinolytischen oder fibrinogenolytischen Substanzen auftretenden lokalen oder systemischen Nebenwirkungen, insbesondere in Form von Blutungen oder einer Hemmung der Blutgerinnung, zu verringern oder zu vermeiden und ein pharmakologisch wirksames Mittel bereitzustellen, mit dem postoperative Adhäsionen verhindert oder verringert werden können.

Diese Aufgabe wird gelöst durch ein rekombinantes Fusionsprotein mit den Merkmalen des Anspruchs 1. Bevorzugte Ausführungsformen finden sich in den Unteransprüchen wieder.

Die Erfinder haben überraschenderweise festgestellt, dass durch die Kopplung mit einer hochmolekularen inerten Stabilisierungsdomäne die unerwünschten Eigenschaften von bekannten fibrinogenolytischen Enzymen zur Prophylaxe postoperativer Adhäsionen, insbesondere im Peritonealraum, vermindert oder verhindert werden können. Die erfindungsgemäßen rekombinanten Fusionsproteine ermöglichen eine präventive Behandlung von Adhäsionen bei Geweben oder Organen, insbesondere peritonealen Adhäsionen nach operativen Eingriffen oder Verletzungen. Bei dem Fusionsprotein wird als biologisch aktive Domäne ein fibrinogenolytisches Enzym oder Enzymfragment an eine hochmolekulare inerte Stabilisierungsdomäne gekoppelt, wodurch ein Fusionsprotein entsteht, welches eine hochmolekulare Fibrinogenase mit neuartigen enzymatischen und pharmakokinetischen Eigenschaften darstellt. Durch die Kopplung des fibrinogenolytischen Enzyms an die hochmolekulare inerte Stabilisierungsdomäne wird das Molekulargewicht signifikant vergrößert sowie eine unspezifische Bindung an die den Peritonealraum auskleidende Mesotheloberfläche erreicht. Überraschenderweise wird hierdurch der Transport durch die Peritonealmembran und damit der Übertritt in den Blutkreislauf verringert. Zudem wird die Verweildauer und damit Wirkungszeit der Fibrinogenase als rekombinanter Wirkstoff in der Bauchhöhle verlängert, wodurch sich der Heilungserfolg bei Applikation an betroffenen Patienten (z.B. Mensch oder Tier) schneller einstellt. Aufgrund der längeren Verweildauer und Wirkungszeit können die notwendigen biologisch aktiven Dosen zum Fibrinogenabbau drastisch reduziert werden, wodurch die anfangs für fibrinolytisch oder fibrinogenolytisch aktiven Substanzen beschriebenen Nachteile in Bezug auf auftretenden Blutungen oder die Hemmung der Blutgerinnung nahezu vollständig eliminiert oder zumindest signifikant reduziert werden können.

Vorzugsweise handelt es sich bei der hochmolekularen inerten Stabilisierungsdomäne um ein Protein, Polypeptid oder Peptid, das ein Molekulargewicht von mehr als 50 kDa, vorzugsweise ein Molekulargewicht von mehr als 80 kDa aufweist. Bevorzugte Molekulargewichte der für die Kopplung an die Enzymdomäne vorgesehenen Stabilisierungsdomäne liegen zwischen 50 und 150 kDa, vorzugsweise zwischen 50 und 100 kDa. Das gewünschte erhöhte Molekulargewicht der Stabilisierungsdomäne kann auch durch Dimerisierung oder Multimerisierung der Stabilisierungsdomäne erzeugt werden, z.B. durch die Dimerisierung eines IgG-Fc Fragments, beispielsweise des humanen IgG1-Fc Fragments.

Die Kopplung der hochmolekularen inerten Stabilisierungsdomäne an das fibrinogenolytische Enzym erfolgt entweder C-terminal und/oder N-terminal. Für die Beibehaltung der enzymatischen Aktivität muss hierbei die Struktur der Enzymdomäne berücksichtigt werden. Gegebenenfalls müssen ausreichend lange Linkersequenzen zwischen den Proteinen eingefügt werden. Falls ein freier N- bzw. C-Terminus für die enzymatische Aktivität erforderlich ist, wird gegebenenfalls nur in einer der möglichen Varianten die enzymatische Aktivität erhalten bleiben. Dies kann der Fachmann anhand bekannter Methoden ohne größeren Aufwand bestimmen, beispielsweise anhand von Aktivitätsassays. In einer bevorzugten Variante können auch mehrere Stabilisierungsdomänen an den C-Terminus oder N-Terminus der Enzymdomäne gekoppelt werden, um diese Erhöhung des Molekulargewichts zu erzielen. Auch eine Kombination unterschiedlicher Stabilisierungsdomänen (z.B. hergestellt als rekombinante Expressionsprodukte) ist möglich. Durch die Kopplung der Stabilisierungsdomäne an das fibrinogenolytische Enzym bleiben die fibrinogenolytischen Eigenschaften des Enzyms auch im Fusionsprodukt erhalten, so dass das resultierende neuartige rekombinante Fusionsprotein bei gleichzeitiger Vermeidung der genannten Nachteile pharmakologisch wirksam ist.

Eine Kopplung von Ancrod an den N-Terminus der Stabilisierungsdomäne hat sich als besonders wirksam herausgestellt. Insbesondere konnte eine im Vergleich zum nativen Ancrod-Molekül verlängerte Verweildauer des Konstrukts im Peritonealraum verzeichnet werden.

Die biologisch aktive Domäne des rekombinanten Fusionsproteins bewirkt den enzymatischen Abbau von Fibrinogen und verhindert damit die Entstehung von Fibrin, der Vorstufe von Adhäsionen. Durch die Kopplung des Enzyms an eine auf Proteinen oder Peptiden basierende Stabilisierungsdomäne wird ein Austritt der biologisch wirksamen Enzymkomponenten aus der Bauchhöhle in das Blutkreislaufsystem vermindert oder verhindert, wodurch die Wirkung auf die lokale, überschießende Fibrinbildung limitiert und eine unerwünschte Hemmung der Blutgerinnung vermindert oder verhindert wird. Dadurch werden Blutungen verhindert, ohne die Heilung der Operationswunde zu beeinträchtigen. Durch die höhere Verweildauer des erfindungsgemäßen Konstruktes wird zudem die Verweildauer der Wirksubstanz erhöht, so dass die Bildung von Adhäsionen über die kritische Heilungsphase von 2 bis 4 Tagen unterbunden wird.

Aus diesen Gründen sind die erfindungsgemäßen rekombinanten Fusionsproteine hervorragende Kandidaten für eine therapeutische Verwendung, da sie aufgrund der geringen systemischen Verfügbarkeit wenig oder keine Nebenwirkungen im Vergleich zu den bisher getesteten Substanzen verursachen. Ferner weisen die erfindungsgemäßen Substanzen aufgrund der langen Wirkdauer eine signifikant bessere Wirksamkeit auf, so dass sie sich generell für alle therapeutischen Anwendungen eignen, die eine längere Wirkdauer der aktiven Substanzen voraussetzen.

In einer bevorzugten Ausführungsform werden die für das fibrinogenolytische Enzym kodierende Domäne und die Stabilisierungsdomäne direkt über deren C-Terminus oder N-Terminus miteinander verknüpft. Die Kopplung der Stabilisierungsdomäne an die Aminosäuresequenz des fibrinogenolytischen Enzyms kann beispielsweise durch Methoden erfolgen, wie sie in der US 2009/0175893A und der US 2014/0017273A beschrieben sind.

Die biologische Aktivität des durch Kopplung eines fibrinogenolytischen Enzyms an eine Stabilisierungsdomäne erzeugten rekombinanten Fusionsproteins kann jedoch durch einen zusätzlichen Linker optimiert werden, der zwischen der Stabilisierungsdomäne und der Enzymdomäne angeordnet ist. In einer bevorzugten Ausführungsform wird daher die inerte Stabilisierungsdomäne über einen variablen Linker mit dem fibrinogenolytischen Enzym verbunden. Die Kopplung des Linkers erfolgt entweder über den C-Terminus oder den N-Terminus der Stabilisierungsdomäne oder der enzymatisch aktiven Domäne. Für die Verknüpfung des Linkers können im Stand der Technik bekannte Standardverfahren eingesetzt werden.

Bei dem Linker handelt es sich vorzugsweise um ein Peptid oder Polypeptid, dessen Aminosäuresequenz eine unterschiedliche Länge oder Verzweigungsgrad aufweisen kann. Ein bevorzugter Linker umfasst beispielsweise sich wiederholende Folgen von Glycin-, Alanin- und Serinresten. Vorzugsweise umfasst der Linker eine Sequenz (GGGGGS)x oder (GGGGA)xR, wobei A = Alanin; G = Glycin; S = Serin; R = Arginin; x = Anzahl der Wiederholungen > 1. Die Anzahl der Wiederholungen x in der Linkersequenz beträgt vorzugsweise zwischen 1 und 4. Der Linker vergrößert den sphärischen Abstand zwischen dem fibrinogenolytischen Enzym und der inerten Stabilisierungsdomäne des erfindungsgemäßen rekombinanten Fusionsproteins.

Neben den oben genannten Peptid-Linkern sind für die Kopplung der beiden Domänen auch an sich bekannte chemische Linker im Sinne der vorliegenden Erfindung einsetzbar, wobei deren Struktur und Länge auf die Orientierung der beiden Domänen im Hinblick auf eine optimierte Expression und enzymatische Aktivität des Fusionsproteins im Rahmen der üblichen Methodik modifizierbar sind.

Die erfindungsgemäßen Konstrukte werden nach ihrer Herstellung auf ihre biologische Aktivität in einem der bekannten Assays überprüft, bei dem die fibrinogenolytische Enzymaktivität der Fusionsproteine ermittelt wird. Die Erfindung umfasst insbesondere solche Fusionsproteine, welche ausreichend fibrinogenolytisch aktiv sind. Zur Optimierung der biologischen Aktivität kann es notwendig sein, die Art und Position der Kopplung, den Aufbau und die Länge des Linkers sowie die Art und den Aufbau der Stabilisierungsdomäne anzupassen. Insbesondere kann eine sterische Behinderung der Stabilisierungsdomäne die Enzymaktivität nachteilig beeinflussen, was jedoch leicht durch einen Fachmann mit Hilfe der zur Verfügung stehenden Mitteln *in-vitro* zu ermitteln ist.

Die erfindungsgemäßen hochmolekularen rekombinanten Fusionsproteine stellen somit hochwirksame Fibrinogenasen dar, welche über Standardmethoden kloniert und in geeigneten Expressionssystemen exprimiert werden können. Aufgrund des relativ hohen Molekulargewichtes der Konstrukte und ihrer komplexen Struktur, welche Disulfidbrücken umfasst, sind eukaryotische Expressionssysteme bevorzugt.

Der hier verwendete Begriff "fibrinogenolytisches Enzym" umfasst Enzyme, aktive Enzymfragmente oder enzymatisch wirksame Substanzen, die eine Fibrinogenase-Aktivität aufweisen und einen Abbau von Fibrinogen bewirken oder fördern. Vorzugsweise handelt es sich bei dem fibrinogenolytischen Enzym der Fibrinogenasedomäne um eine Serinprotease, vorzugsweise eine thrombinähnliche Serinprotease. Bevorzugte fibrinogenolytische Enzyme sind beispielsweise die aus Schlangengiften isolierten Enzyme Ancrod oder Batroxobin. Diese Enzyme haben sich zur Kopplung mit der hochmolekularen inerten Stabilisierungsdomäne als sehr geeignet herausgestellt, da sie ihre Aktivität in dem erfindungsgemäßen rekombinanten Fusionsprotein zumindest weitgehend erhalten. Daneben gibt es weitere geeignete Kandidaten, beispielsweise thrombinähnliche, aus Schlangengiften isolierte, Proteasen und rekombinante Versionen davon. Insbesondere sind rekombinante Formen oder Varianten der Enzyme Ancrod oder Batroxobin als Fibrinogenasedomäne des rekombinanten Fusionsproteins geeignet.

Bei der hochmolekularen inerten Stabilisierungsdomäne handelt es sich vorzugsweise um Serumalbumin, vorzugsweise tierisches oder humanes Serumalbumin. Bei einer weiteren Variante umfasst die hochmolekulare inerte Stabilisierungsdomäne Transferrin oder durch genetische Modifikationen inaktivierte Varianten von Transferrin. Bei einer weiteren Variante umfasst die hochmolekulare inerte Stabilisierungsdomäne künstliche Aminosäuresequenzen, wie z.B. PAS (Schlapschy et. al 2013) oder XTEN (US 2013/0165389 A1). Ferner können auch Antikörper oder Antikörperfragmente als Stabilisierungsdomäne mit dem fibrinogenolytischen Enzym gekoppelt werden. Zur prophylaktischen oder therapeutischen Applikation bei Menschen werden als Antikörper vorzugsweise monoklonale Antikörper, humanisierte Antikörper oder Antikörperfragmente von diesen verwendet. Ferner können auch Varianten der erwähnten Stabilisierungsdomänen für den erfindungsgemäßen Zweck verwendet werden. Weitere Ausführungsformen sehen ferner den Einsatz synthetischer Domänen oder anderer inerter Proteindomänen vor, soweit sie im Stand der Technik beschrieben sind.

In einer bevorzugten Variante umfasst das Fusionsprotein eine Aminosäuresequenz, welche für das Enzym Ancrod und eine Stabilisierungsdomäne (z.B. Serumalbumin oder ein IgG-Fc Antikörperfragment) umfasst. Vorzugsweise umfasst das Fusionsprotein eine wie in SEQ ID NO. 2 oder SEQ ID NO. 4 wiedergegebene Aminosäuresequenz oder fibrinogenolytisch wirksame Teile dieser Sequenz. Fibrinogenolytisch wirksame Teile dieser Sequenz sind Sequenzabschnitte, die für die Enzymdomäne und die Stabilisierungsdomäne codieren und enzymatisch beim Fibrinogenabbau wirksam sind. In den konkreten Beispielen wurden solchen fibrinogenolytischen Teilen beispielsweise noch ein Hexahistidin-Tag (His-Tag) zur einfacheren Aufreinigung und ein Signalpeptid angefügt. Ferner können zusätzliche oder alternative Aminosäuren an die oben genannten Sequenzen angefügt oder entfernt sein.

Die erfindungsgemäßen Fusionsproteine sind fibrinogenolytisch hochwirksam und führen zu einem spezifischen Abbau von Fibrinogen. Als sekundärer Effekt ist bei Applikation im Organismus ferner auch eine fibrinolytische Wirkung nachweisbar, da die Abbauprodukte des Enzyms, desA-profibrin und desAA-Fibrinmonomere, lösliche Fibrinkomplexe bilden, die wiederum über die Stimulation des endogenen t-PA zu einer Plasminogen-Aktivierung führen. Dieser Effekt ist als Zunahme der Plasmin-Konzentration nach Applikation des Enzyms in vivo messbar. Dieser Effekt bietet einen weiteren Vorteil, da hohe Konzentrationen des Plasminogenaktivator-Inhibitors (PAI-1) bei Verletzungen zum Versagen des natürlichen Fibrinabbaus und zur Adhäsion führen.

Durch die C-terminale Kopplung von Ancrod oder einem anderen fibrinogenolytisch-aktiven Protein an eine Stabilisierungsdomäne, beispielsweise an Serumalbumin oder ein IgG-Fc Antikörperfragment, wird die Verweildauer des Konstrukts im Peritonealraum im Vergleich zu dem nativen fibrinogenolytisch-aktiven Enzym deutlich verlängert. Das Konstrukt kann auf diese Weise eine sehr viel längere Wirkungsdauer entfachen, als dies bei einem nativen Ancrod-Molekül der Fall wäre. Durch die Retention des Konstrukts im Peritonealraum wurde auch der Übertritt der aktiven Substanz in den Blutkreislauf deutlich verringert.

Die vorliegende Erfindung betrifft ferner eine pharmazeutische Zusammensetzung, die ein wie oben beschriebenes rekombinantes Fusionsprotein umfasst. Die pharmazeutische Zusammensetzung umfasst einen pharmazeutisch annehmbaren Träger und kann als Lösung direkt in den betroffenen Wundraum, beispielsweise den Bauchraum, appliziert werden. Dadurch verhindert der Wirkstoff die Entstehung von Adhäsionen im gesamten Kompartiment, insbesondere dort, wo potentiell Verletzungen aufgetreten sein können. Aufgrund des hohen Molekulargewichtes verbleibt das rekombinante Fusionsprotein als aktive Substanz für einen im Vergleich zu nichtmodifizierten natürlichen Substanzen längeren Zeitraum in dem Peritonealraum und tritt nicht bzw. nur in einem geringen Umfang in das Blutkreislaufsystem ein. Dadurch werden beispielsweise systemische Effekte und Nebenwirkungen der natürlichen Substanz (beispielsweise von Ancrod oder Batroxobin) verhindert oder verringert. Die aktive Substanz kann während oder nach der Operation einmalig oder mehrmals in die Peritonealhöhle appliziert werden. Eine einmalige Applikation ist bevorzugt, so dass weitere Injektionen oder Infusionen nicht zwingend erforderlich sind. Dieser Vorteil ist bedingt durch die im Vergleich zu den natürlichen Substanzen längere Verweildauer des rekombinanten Konstruktes in der Bauchhöhle.

In einer bevorzugten Variante kann das erfindungsgemäße rekombinante Fusionsprotein in Kombination mit anderen Behandlungsmethoden oder Produkten verwendet werden. So ist beispielsweise eine Kombination des Fusionsproteins mit physikalischen Barrieremethoden aus festen oder flüssigen Membranen, Gelen oder Sprays denkbar. Vorzugsweise sind diese biologisch abbaubar.

Durch die Verwendung speziesspezifischer Domänen als Stabilisierungsdomäne (beispielsweise humanem Serumalbumin (HSA) oder ein humanes IgG-Fc Antikörperfragment) weist das erfindungsgemäße rekombinante Fusionsprotein eine geringe Immunogenität und keine eigene pharmakodynamische Wirkung auf. Aufgrund des hohen Molekulargewichtes von vorzugsweise > 50 kDa wird zudem eine lange biologische Haltbarkeit gewährleistet.

Um eine optimale prophylaktische Wirksamkeit der erfindungsgemäßen pharmazeutischen Zusammensetzung zu entfalten, muss die fibrinogenolytische Wirkung während der gesamten Zeit der Wundheilung vorliegen. Vorzugsweise liegt daher das rekombinante Fusionsprotein am Wirkort in einer effektiven Konzentration vor. Der für die vollständige Wundheilung erforderliche Zeitraum beträgt zwischen 1 und 8 Tagen, für die prophylaktische Behandlung wird ein Zeitraum von 2 bis 4 Tagen bevorzugt.

Die für die vorbeugende oder therapeutische Wirksamkeit erforderliche enzymatische Aktivität der in der in die Bauchhöhle applizierten pharmazeutisch wirksamen Lösung liegt im Bereich zwischen 0,01 und 10 Units/ml über den gesamten Zeitraum der Wundheilung. Vorzugsweise werden Konzentrationen des Fusionsproteins zwischen 0,1 bis 5 Einheiten/ml (Units/ml) eingesetzt. Vorzugsweise wird das rekombinante Fusionsprotein in einem osmotisch aktiven Medium (z.B. Icodextrinlösung) angewendet.

Die vorliegende Beschreibung betrifft ferner eine kombinierte Anwendung des Fusionsproteins mit anderen Produkten zur Vorbeugung von Gewebeverklebungen (Adhäsionen), insbesondere nach operativen Eingriffen. Hierfür können beispielsweise Membranen eingesetzt werden, die aus oxidierter regenerierter Zellulose, Polytetrafluorethylen, Hyaluronsäure-Carboxymethylcellulose oder Polyethylenglycol bestehen. Ferner können flüssige Adhäsionsbarrieren eingesetzt werden, welche die Organe und Gewebe mittels Hydroflotation trennen. Vorzugsweise kommen hierbei Hyaluronsäure, quervernetzte Hyaluronsäure oder Icodextrin zum Einsatz.

Das erfindungsgemäße rekombinante Fusionsprotein ist vorzugsweise in einer biokompatiblen, bioabbaubaren Matrix eingebettet, die das Fusionsprotein während der initialen Heilungsphase, vorzugsweise über einen Zeitraum von 2 bis 4 Tagen, kontinuierlich freisetzt.

### Wege zur Ausführung der Erfindung:

Die Erfindung wird in den nachfolgenden Beispielen erläutert.

### Beispiele:

### Beispiel 1:

### Herstellung von N-Ancrod-Fc-Fusionsprotein

Für die Herstellung der erfindungsgemäßen Zusammensetzung wurde ein Fusionsprotein hergestellt, bestehend aus Ancrod und der konstanten Region eines humanen IgG1 Antikörpers. Zwischen der biologisch aktiven Ancrod-Domäne und der durch das IgG1-Fc-Antikörperfragment gebildeten Stabilisierungsdomäne ist ein Glycin-Alanin-Linker eingefügt. Zur Verbesserung der Sekretion ins Zellkulturmedium und zur leichteren Aufreinigung wurde N-terminal das Signalpeptid des humanen Serumalbumins angefügt.

Für die Herstellung wurde die Sequenz des Ancrod-Proteins (Zugangsnummer: ABN13428.1) C-terminal mit der konstanten Region eines humanen IgG1 (Uniprot acc.no. P01857-1, Aminosäuren 104 - 330) über einen flexiblen Glycin-Alanin-Linker an dessen C-Terminus fusioniert. Anschließend wurden das für die Aufreinigung vorgesehene HSA-Signalpeptid (Aminosäuren 1 bis 18) angefügt. Für die Synthese der für das Fusionsprotein kodierenden cDNA wurden die DNA-Codons für die Expression in humanen Zellen optimiert. Am 5' Ende der cDNA wurden die Restriktionsschnittstellen für Notl und Xbal und am 3' Ende für BstXI und HindIII angefügt, die die Klonierung der DNA in die entsprechenden Vektoren für eine transiente Expression bzw. die für die Herstellung von stabilen Zelllinien erlaubt. Das resultierende cDNA-Konstrukt wurde synthetisch hergestellt.

Diese cDNA wurde kloniert, amplifiziert und in einen Expressionsvektor für die transiente Transfektion umkloniert. Die korrekte Insertion der cDNA wurde über einen Restriktionsverdau geprüft. Mit dem resultierenden Plasmid wurden anschließend E. coli Bakterien (DH5α) transformiert und der Stamm in 0,8 Liter LB Medium kultiviert. Daraus wurde die Plasmid-DNA endotoxinfrei isoliert und die Lösung steril filtriert.
Für die transiente Expression des Proteins wurden HEK-F Zellen in serumfreier Suspensionskultur in einem Volumen von 500 ml im Schüttelkolben (ca. 2,5x10⁶ Zellen/ml) angesetzt. Die Transfektion der Zellen erfolgte über ein verzweigtes PEG-Aminoester-Copolymer mit einem Transfektionsansatz von ca. 10 µg DNA/1x10⁷ Zellen - DNA/coPEG33 - 1/6(w/w). Nach Zugabe von Valproinsäure wurde die Zellkultur für weitere 7 Tage kultiviert. Danach wurde der Zellkulturüberstand durch Zentrifugation geerntet. Die Chromatographie erfolgte in 50 mM MES Puffer, pH 5.5.

Die Reinigung des Fusionsproteins erfolgte durch Ionenaustauscher-Chromatographie. Als Säulenmaterial wurde HiTrap SP FF Affinity Resin (GE Healthcare Europe GmbH, Freiburg, Germany) verwendet. Die Elution des Fusionsproteins erfolgte durch einen Kochsalzgradienten. Die Analyse der Eluatfraktionen erfolgte durch Gelelektrophorese (SDS-PAGE) und geeignete Proteinfraktionen wurden gepoolt und gegen gepufferte Saline (PBS) dialysiert, aliquotiert und bis zur weiteren Verwendung bei -20°C gelagert.

Die cDNA-Sequenz des Konstruktes ist in der SEQ ID NO 1 gezeigt:
Unterstrichen sind die eingefügten Restriktionsschnittstellen. - in Fettschrift ist die für das Fusionsprotein codierende Sequenz gezeigt.

Die daraus resultierende Aminosäuresequenz ist in der SEQ ID NO 2 gezeigt:

Die Aminosäuresequenz beginnt mit dem Signalpeptid des humanen Serumalbumins MKWVTFISLLFLFSSAYS (unterstrichen dargestellt), welches bei der Sekretion des Proteins von der Zelle abgespalten wird. Der zwischen dem humanen Serumalbumin und der Ancrod-Domäne angeordnete Linker GGGGAGGGGAGGGGAR ist mit dem C-Terminus des Ancrods verbunden (in Fettschrift dargestellt).

### Beispiel 2:

### Herstellung von N-Ancrod-HSA-C-Fusionsprotein mit His-Tag

In diesem Beispiel wird eine weitere Variante eines auf Ancrod basierenden Fusionsproteins gezeigt, bestehend aus Ancrod, humanem Serumalbumin (HSA), einem Signalpeptid des humanen Serumalbumins und einem nachfolgenden His-Tag. Zwischen der biologisch aktiven Ancrod-Domäne und der durch das humane Serumalbumin gebildeten Stabilisierungsdomäne ist ein Glycin-/Serinlinker eingefügt.

Für die Herstellung wurde die Sequenz des Ancrod-Proteins (Zugangsnummer: ABN13428.1) C-terminal mit dem N-Terminus des humanen Serumalbumins (HSA) (Zugangsnummer: P02768, Aminosäuren 25-609) fusioniert. Anschließend wurde das HSA-Signalpeptid (Aminosäuren 1 bis 18) angefügt. Die cDNA wurde wie oben beschrieben prozessiert und das Protein exprimiert.

Die cDNA-Sequenz des Konstruktes ist in der SEQ ID NO 3 gezeigt: Unterstrichen sind die eingefügten Restriktionsschnittstellen (5': NotI, XbaI; 3': BstXI, HindIII) - in Fettschrift ist die für das Fusionsprotein codierende Sequenz gezeigt.

Die daraus resultierende Aminosäuresequenz ist in der SEQ ID No 4 gezeigt:

Die Aminosäuresequenz beginnt mit dem Signalpeptid des humanen Serumalbumins MKWVTFISLLFLFSSAYS, welches bei der Sekretion des Proteins von der Zelle abgespalten wird. Der zwischen dem humanen Serumalbumin und der Ancrod-Domäne angeordnete Linker GGGGSGGGGSGGGGS ist mit dem N-Terminus des Serumalbumins verbunden (in Fett dargestellt).

### Beispiel 3:

### Aktivitätsbestimmung der hergestellten Fusionsproteine auf ihre fibrinogenolytische Enzymaktivität

Die Aktivitätsbestimmung der Fusionsproteine erfolgte mit Fibrinogen als Substrat (1 mg/ml) gelöst in 10 mM Tris-HCI, 0.15 M NaCl, pH 7.4. Jeweils 500 µl der Fibrinogen Lösung wurden in eine Küvette pipettiert. Nach 2 Minuten wurden 100 µl Probe oder eine Positivkontrolle (Batroxobin) hinzugefügt. Danach wurde die Messung der Trübungszunahme bei 340 nm über einen Zeitraum von einer Stunde photometrisch bestimmt und die maximale Steigung der Kurve bestimmt. Die maximale Steigung der Kurve ist proportional der enzymatischen Aktivität, die anhand einer Eichkurve in Units/ml umgerechnet wird.

### Beispiel 4:

### Behandlung von peritonealen Adhäsionen bei Säugetieren

Zur präventiven oder therapeutischen Applikation in einem Säugetier (z. B. einem Menschen oder einem Versuchstier) wird das nach der Expression isolierte und aufgereinigte erfindungsgemäße rekombinante Fusionsprotein oder ein entsprechendes Placebo nach dem Adhäsionen auslösenden chirurgischen Eingriff direkt in die Bauchhöhle des Versuchstiers appliziert. Für eine optimale Wirkung wird eine enzymatische Aktivität zwischen 0,01 und 10 U/ml angestrebt. Die pharmazeutische Lösung umfasst das Fusionsprotein mit einer Aktivität zwischen 0,1 bis 5 U/ml.

Nach Applikation des Fusionsproteins wird eine über mehrere Tage anhaltende fibrinolytische Enzymaktivität des Fusionsproteins bei gleichzeitiger Aufrechterhaltung der Wundheilung feststellbar sein. Im Vergleich zu den mit Placebo behandelten Versuchstieren wird der Anteil und Schweregrad der im Rahmen des Wundheilungsprozesses auftretenden Verwachsungen drastisch reduziert sein.

### Beispiel 5:

### Pharmakologische und pharmakokinetische Eigenschaften von N-Ancrod-HSA-C-Fusionsproteinen

Für die Herstellung des Fusionsproteins wurde die Sequenz des Ancrod-Proteins (Zugangsnummer: ABN13428.1) C-terminal mit dem N-Terminus des humanen Serumalbumins (HSA) (Zugangsnummer: P02768, Aminosäuren 25-609) fusioniert. Die enzymatische Aktivität des Fusionsproteins beträgt 24 U/ml.

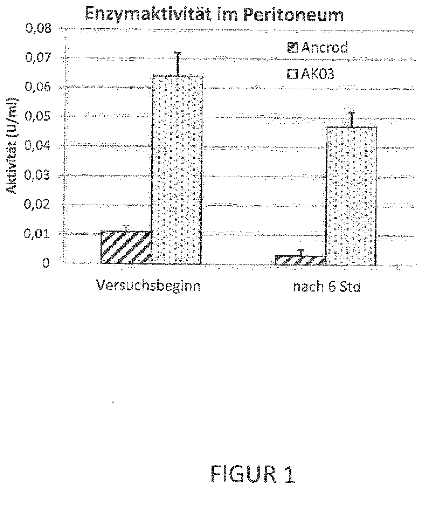

Da natives Ancrod für eine therapeutische Anwendung zur Behandlung peritonealer Adhäsionen ungeeignet ist, wurde das so erhaltene Fusionsprotein auf dessen Aktivität und Verbleib (Verweildauer) in der Bauchhöhle getestet. In Fig. 1 ist die Enzymaktivität in der Flüssigkeit in der Bauchhöhle nach einer einmaligen intraperitonealen Applikation von nativem Ancrod und einem erfindungsgemäßen Ancrod-Fusionsprotein (AK03) gezeigt. Deutlich ist zu erkennen, dass das native Ancrod für eine therapeutische Anwendung ungeeignet ist, da das intraperitoneal verabreichte Ancrod sehr schnell die Bauchhöhle verlässt und damit nur geringe Konzentrationen erreicht werden, die zudem innerhalb von 6 Stunden zu Werten nahe der Nachweisgrenze abfallen. Im Gegensatz dazu werden nach Applikation einer vergleichbaren Dosis des Ancrod-Fusionsproteins (AK03) wesentlich höhere Aktivitäten in der Bauchhöhle erreicht, die auch nach 6 Stunden noch im therapeutisch wirksamen Bereich liegen.

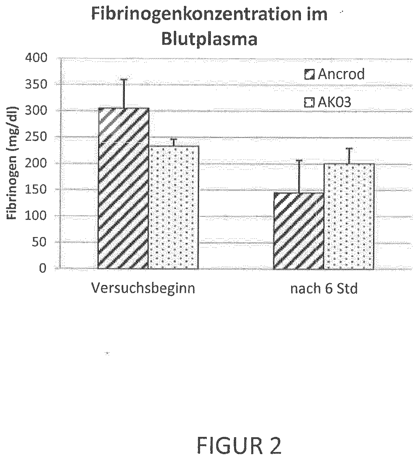

In Fig. 2 ist die Wirkung von Ancrod und des erfindungsgemäßen Ancrod-Fusionsproteins (AK03) auf die Fibrinogenkonzentration im Blutplasma gezeigt. Der schnelle Übertritt von Ancrod in das Blutgefäßsystem führt hier zu einem Abfall des Fibrinogen-Spiegels im Blut um mehr als 50%. AK03 tritt aufgrund der geänderten Molekülstruktur nur sehr langsam in den Blutkreislauf über und führt daher nur zu einem geringen Abfall des Fibrinogen-Spiegels von 14 %. Derart geringe Änderungen der Fibrinogen Konzentration liegen im physiologischen Bereich und haben keine Auswirkungen auf die Blutgerinnung.

Das mit der Stabilisierungsdomäne versehene Ancrod-Fusionsprotein AK03 zeigt damit ein weitaus günstigeres pharmakokinetisches Verhalten als das native Ancrod-Molekül.

Diese Ergebnisse zeigen, dass das Ancrod-Fusionsprotein ähnliche enzymatische Eigenschaften wie Ancrod aufweist, sich aber pharmakokinetisch deutlich unterscheidet. Insbesondere führt dies zu einer längeren Verweildauer des Fusionsproteins im Peritonealraum und einem geringeren Übertritt der Substanz in den Blutkreislauf. Durch diese Eigenschaften ist das erfindungsgemäße Fusionsprotein besonders geeignet für die intraperitoneale Applikation für die Behandlung/Vorbeugung peritonealer Adhäsionen.

### Material & Methoden

### Pharmakokinetik am Hund

3 Beagle-Hunde wurden mit venösen und intraperitonealen Verweilkathetern versehen. Eine Woche nach Katheterimplantation erhielten die Tiere eine einmalige intraperitoneale Injektion der Prüfsubstanz. 0,5 ml Proben der Peritonealflüssigkeit wurden 0, 0,5; 1, 2, 4, 6, und 8 Stunden nach Substanzapplikation entnommen, venöse Blutproben zur Gewinnung von Citratplasma zu den Zeitpunkten 0, 0,5; 1, 2, 4, 6, 8, 16 und 24 Stunden. Die Fibrinogenkonzentration in den Plasmaproben wurde mit der Methode nach Clauss photometrisch gemessen. Die Enzym Aktivität in der Peritonealflüssigkeit wurde nach Zentrifugation der Proben mit Hilfe einer kinetischen Methode turbidimetrisch nach Zugabe von humanem Fibrinogen bestimmt.

### Pharmakokinetik an der Ratte

Sprague Dawley Ratten erhielten erhielten in kurzer Inhalationsnarkose intraperitoneale Injektionen der Prüfsubstanz. Gleichzeitig wurde eine venöse Blutprobe entnommen. Danach wurden die Tiere in den Käfig zurückgesetzt wo sie nach kurzer Zeit erwachten. 6 Stunden nach Substanzapplikation wurden die Tiere erneut narkotisiert und die Peritonealflüssigkeit und eine weitere Blutprobe zur Gewinnung von Citratplasma entnommen. Beide Proben wurden sofort nach Entnahme zentrifugiert, bei -80°C schockgefroren und zu einem späteren Zeitpunkt mit den oben beschriebenen Methoden analysiert.

### Literaturverzeichnis

Arung, W.; Meurisse, M.; Detry, O. (2011): Pathophysiology and prevention of postoperative peritoneal adhesions. In: World J Gastroenterol 17 (41), S. 4545-4553.
Ashby, E. C.; James, D. C.; Ellis, H. (1970): The effect of intraperitoneal Malayan pit-viper venom on adhesion formation and peritoneal healing. In: Br J Surg 57 (11), S. 863.
Binda, M. M.; Hellebrekers, B. W.; Declerck, P. J.; Koninckx, P. R. (2009): Effect of Reteplase and PAI-1 antibodies on postoperative adhesion formation in a laparoscopic mouse model. In: Surgical Endoscopy 23 (5), S. 1018-1025.
Binnebösel, M.; Klink, C.; Serno, J.; Jansen, P.; Trotha, K. von; Neumann, U.; Junge, K. (2011): Chronological evaluation of inflammatory mediators during peritoneal adhesion formation using a rat model. In: Langenbeck's Archives of Surgery 396 (3), S. 371-378.
Broek, R. P. G. ten; Issa, Y.; van Santbrink, E. J. P.; Bouvy, N. D.; Kruitwagen, R. F. P. M.; Jeekel, J. et al. (2013a): Burden of adhesions in abdominal and pelvic surgery: systematic review and met-analysis. In: BMJ 347, S. f5588.
Broek, R. P. G. ten; Kok-Krant, N.; Bakkum, E. A.; Bleichrodt, R. P.; van Goor, H. (2013b): Different surgical techniques to reduce post-operative adhesion formation: a systematic review and meta-analysis. In: Hum Reprod Update 19 (1), S. 12-25.
Brokelman, W. J. A.; Lensvelt, M.; Borel Rinkes, I. H. M.; Klinkenbijl, J. H. G.; Reijnen, M. M. P. J. (2011): Peritoneal changes due to laparoscopic surgery. In: Surg Endosc 25 (1), S. 1-9.
Brummer, T. H. I.; Jalkanen, J.; Fraser, J.; Heikkinen, A-M.; Kauko, M.; Mäkinen, J. et al. (2011): FINHYST, a prospective study of 5279 hysterectomies: complications and their risk factors. In: Hum Reprod 26, (7) S. 1741-1751.
Buckman, RFJr; Bordos, D.; Bell, W. R.; Cameron, J. L. (1975): Prevention of experimental postoperative adhesions by ancrod defibrinogenation. In: J Surg Res 18 (4), S. 377-384.
Chowdhury, S. M.; Hubbell, J. A. (1996): Adhesion prevention with ancrod released via a tissue-adherent hydrogel. In: J Surg Res 61 (1), S. 58-64.
Cohen, Z.; Senagore, A. J.; Dayton, M. T.; Koruda, M. J.; de Beck; Wolff, B. G. et al. (2005): Prevention of postoperative abdominal adhesions by a novel, glycerol/sodium hyaluronate/carboxymethylcellulose-based bioresorbable membrane: a prospective, randomized, evaluator-blinded multicenter study. In: Diseases of the colon and rectum 48 (6), S. 1130-1139.
Diamond, M. P.; Kruger, M.; Saed, G. M. (2004): Effect of Tisseel on expression of tissue plasminogen activator and plasminogen activator inhibitor-1. In: Fertility and sterility 81 (6), S. 1657-1664.
Ellis, H.; Moran, B. J.; Thompson, J. N.; Parker, M. C.; Wilson, M. S.; Menzies, D. et al. (1999): Adhesion-related hospital readmissions after abdominal and pelvic surgery: a retrospective cohort study. In: Lancet 353 (9163), S. 1476-1480.
Fossum, G. T.; Silverberg, K. M.; Miller, C. E.; Diamond, M. P.; Holmdahl, L. (2011): Gynecologic use of Sepraspray Adhesion Barrier for reduction of adhesion development after laparoscopic myomectomy: a pilot study. In: Fertil Steril 96 (2), S. 487-491.
Hellebrekers, B. W. J.; Emeis, J. J.; Kooistra, T.; Trimbos, J. B.; Moore, N. R.; Zwinderman, K. H.; Trimbos-Kemper, T. C. (2005): A role for the fibrinolytic system in postsurgical adhesion formation. In: Fertil Steril 83 (1), S. 122-129.
Hellebrekers, B. W.; Trimbos-Kemper, T. C.; Boesten, L.; Jansen, F. W.; Kolkman, W.; Trimbos, J. B. et al. (2009): Preoperative predictors of postsurgical adhesion formation and the Prevention of Adhesions with Plasminogen Activator (PAPA-study): results of a clinical pilot study. In: Fertil Steril 91 (4), S. 1204-1214.
Hellebrekers, B. W. J.; Kooistra, T. (2011): Pathogenesis of postoperative adhesion formation. In: Br J Surg 98 (11), S. 1503-1516.
Hill-West, J. L.; Dunn, R. C.; Hubbell, J. A. (1995): Local release of fibrinolytic agents for adhesion prevention. In: J Surgical Research 59 (6), S. 759-763.
Klingler, P. J.; Floch, N. R.; Seelig, M. H.; Branton, S. A.; Wolfe, J. T.; Metzger, P. P. (1999): Seprafilm-induced peritoneal inflammation: a previously unknown complication. Report of a case. In: Diseases of the colon and rectum 42 (12), S. 1639-1643.
Kössi, J.; Salminen, P.; Rantala, A.; Laato, M. (2003): Population-based study of the surgical workload and economic impact of bowel obstruction caused by postoperative adhesions. In: Br J Surg 90 (11), S. 1441-1444.
Parker, M. C.; Wilson, M. S.; Menzies, D.; Sunderland, G.; Clark, D. N.; Knight, A. D.; Crowe, A. M. (2005): The SCAR-3 study: 5-year adhesion-related readmission risk following lower abdominal surgical procedures. In: Colorectal Dis 7 (6), S. 551-558.
Parker, M. C.; Ellis, H.; Moran, B. J.; Thompson, J. N.; Wilson, M. S.; Menzies, D. et al. (2001): Postoperative adhesions: ten-year follow-up of 12,584 patients undergoing lower abdominal surgery. In: Dis Colon Rectum 44 (6), S. 822-830.
Ray, N. F.; Denton, W. G.; Thamer, M.; Henderson, S. C.; Perry, S. (1998): Abdominal adhesiolysis: inpatient care and expenditures in the United States in 1994. J Am Coll Surg 186 (1), S. 1-9.
Saed, G. M.; Diamond, M. P. (2004): Molecular characterization of postoperative adhesions: the adhesion phenotype. In: The Journal of the American Association of Gynecologic Laparoscopists 11 (3), S. 307-314.
SCAR Group (2013): Pathogenesis, consequences, and control of peritoneal adhesions in gynecologic surgery: a committee opinion. Fertil Steril 99 (6), S. 1550-1555.
Schnüriger, B.; Barmparas, G.; Branco, B. C.; Lustenberger, T.; Inaba, K.; Demetriades, D. (2011): Prevention of postoperative peritoneal adhesions: a review of the literature. In: The American Journal of Surgery 201 (1), S. 111-121.
Schlapschy M, Binder U, Börger C, et al. (2013) PASylation: a biological alternative to PEGylation for extending the plasma half-life of pharmaceutically active proteins. Protein Eng Des Sel. 26(8):489-501.
Sikirica, V.; Bapat, B.; Candrilli, S. D.; Davis, K. L.; Wilson, M.; Johns, A. (2011): The inpatient burden of abdominal and gynecological adhesiolysis in the US. BMC Surg 11, S. 13.
Wallwiener, M.; Koninckx, P. R.; Hackethal, A.; Brölmann, H.; Lundorff, P.; Mara, M. et al. (2014): A European survey on awareness of post-surgical adhesions among gynaecological surgeons. In: Gynecol Surg 11 (2), S. 105-112.

## Patentansprüche

1. Rekombinantes Fusionsprotein umfassend ein fibrinogenolytisches Enzym mit einer Aminosäuresequenz, welche C-terminal und/oder N-terminal mit der Aminosäuresequenz wenigstens einer hochmolekularen inerten Stabilisierungsdomäne mit einem Molekulargewicht > 50 kDa verbunden ist, zur Verwendung bei der Vorbeugung oder Behandlung von peritonealen Adhäsionen nach operativen Eingriffen im Peritonealraum.

2. Rekombinantes Fusionsprotein zur Verwendung nach Anspruch 1, **dadurch gekennzeichnet, dass** die inerte Stabilisierungsdomäne über einen Linker mit dem fibrinogenolytischen Enzym verbunden ist.

3. Rekombinantes Fusionsprotein zur Verwendung nach Anspruch 2, **dadurch gekennzeichnet, dass** es sich bei dem Linker um einen Glycin-Serin-Linker mit der Sequenz (GGGGGS)x oder einen Glycin-Alanin-Linker mit der Sequenz (GGGGA)xR handelt, wobei A = Alanin; G = Glycin; R = Arginin; S = Serin; x = Anzahl der Wiederholungen > 1.

4. Rekombinantes Fusionsprotein zur Verwendung nach Anspruch 1, **dadurch gekennzeichnet, dass** mehrere inerte Stabilisierungsdomänen an den C-Terminus des fibrinogenolytischen Enzyms gekoppelt sind.

5. Rekombinantes Fusionsprotein zur Verwendung nach Anspruch 1, **dadurch gekennzeichnet, dass** mehrere inerte Stabilisierungsdomänen an den N-Terminus des fibrinogenolytischen Enzyms gekoppelt sind.

6. Rekombinantes Fusionsprotein zur Verwendung nach Anspruch 1, **dadurch gekennzeichnet, dass** die Stabilisierungsdomäne ein Molekulargewicht zwischen 50 und 150 kDa aufweist.

7. Rekombinantes Fusionsprotein zur Verwendung nach einem der vorhergehenden Ansprüche, **dadurch gekennzeichnet, dass** es sich bei dem fibrinogenolytischen Enzym um eine thrombinähnliche Serinprotease handelt.

8. Rekombinantes Fusionsprotein zur Verwendung nach einem der vorhergehenden Ansprüche, **dadurch**
**gekennzeichnet, dass** es sich bei dem fibrinogenolytischen Enzym um Ancrod oder Batroxobin, oder eine rekombinante Variante von Ancrod oder Batroxobin handelt.

9. Rekombinantes Fusionsprotein zur Verwendung nach einem der vorhergehenden Ansprüche, **dadurch gekennzeichnet, dass** es sich bei der hochmolekularen inerten Stabilisierungsdomäne um eine Protein- oder Peptiddomäne, Serumalbumin, Transferrin, einen monoklonalen oder humanisierten Antikörper oder ein Antikörperfragment oder um eine künstliche Domäne, z. B. PAS oder XTEN handelt.

10. Rekombinantes Fusionsprotein zur Verwendung nach Anspruch 1, **dadurch gekennzeichnet, dass** die Aminosäuresequenz von humanem Serumalbumin, oder einem Teil dieser Sequenz mit einem Molekulargewicht > 50 kDa, als inerte Stabilisierungsdomäne an den C-Terminus oder N-Terminus von Ancrod oder Batroxobin als fibrinogenolytischem Enzym gekoppelt ist.

11. Rekombinantes Fusionsprotein zur Verwendung nach Anspruch 10, **dadurch gekennzeichnet, dass** das Fusionsprotein eine wie in SEQ ID NO. 2 (N-terminale Fusion) oder SEQ ID NO. 4 (C-terminale Fusion) wiedergegebene Aminosäuresequenz, oder fibrinogenolytisch wirksame Teile dieser Sequenz, umfasst.

12. Rekombinantes Fusionsprotein zur Verwendung nach einem der vorhergehenden Ansprüche, **dadurch gekennzeichnet, dass** das Fusionsprotein zur kontinuierlichen intraperitonealen Freisetzung eingebettet in einer bioabbaubaren Matrix vorliegt.

13. Pharmazeutische Zusammensetzung umfassend ein rekombinantes Fusionsprotein nach einem der Ansprüche 1 bis 12 zur Verwendung bei der Vorbeugung oder Behandlung von peritonealen Adhäsionen nach operativen Eingriffen im Peritonealraum.

14. Pharmazeutische Zusammensetzung zur Verwendung nach Anspruch 13, **dadurch gekennzeichnet, dass** die Zusammensetzung ein osmotisch aktives Medium umfasst, wobei das rekombinante Fusionsprotein in dem osmotisch aktiven Medium, vorzugsweise Icodextrinlösung, vorliegt.

## Claims

1. A recombinant fusion protein comprising a fibrinogenolytic enzyme having an amino acid sequence that is C-terminally and/or N-terminally linked to the amino acid sequence of at least one high-molecular inert stabilization domain with a molecular weight of > 50 kDa, for the prevention or treatment of peritoneal adhesions following surgical interventions in the peritoneal cavity.

2. The recombinant fusion protein for use according to claim 1, **characterized in that** the inert stabilization domain is connected via a linker to the fibrinogenolytic enzyme.

3. The recombinant fusion protein for use according to claim 2, **characterized in that** the linker relates to a glycine-serine linker having the sequence (GGGGGS)x, or glycine-alanine linker with the sequence (GGGGA)xR, where A = alanine; G = glycine; R = arginine; S = serine; x = number of repetitions > 1.

4. The recombinant fusion protein for use according to claim 1, **characterized in that** a plurality of inert stabilization domains are linked to the C-terminus of the fibrinogenolytic enzyme.

5. The recombinant fusion protein for use according to claim 1, **characterized in that** a plurality of inert stabilization domains are linked to the N-terminus of the fibrinogenolytic enzyme.

6. The recombinant fusion protein for use according to claim 1, **characterized in that** the stabilization domain has a molecular weight between 50 and 150 kDa.

7. The recombinant fusion protein for use according to any one of the preceding claims, **characterized in that** the fibrinogenolytic enzyme relates to a thrombin-like serine protease.

8. The recombinant fusion protein for use according to any one of the preceding claims, **characterized in that** the fibrinogenolytic enzyme relates to ancrod or batroxobin, or a recombinant variant of ancrod or batroxobin.

9. The recombinant fusion protein for use according to any one of the preceding claims, **characterized in that** the high molecular inert stabilization domain relates to a protein or peptide domain, in particular serum albumin, transferrin, a monoclonal or humanized antibody or an antibody fragment, or an artificial domain, e.g. PAS or XTEN.

10. The recombinant fusion protein for use according to claim 1, **characterized in that** the amino acid sequence of human serum albumin, or a part of this sequence having a molecular weight of > 50 kDa, is linked as inert stabilizing domain to the C terminus or N-terminus of ancrod or batroxobin as fibrinogenolytic enzyme.

11. The recombinant fusion protein for use according to claim 10, **characterized in that** the fusion protein comprises an amino acid sequence set forth in SEQ ID NO. 2 (N-terminal fusion) or SEQ ID NO. 4 (C-terminal fusion), or fibrinogenolytically effective parts of this sequence.

12. The recombinant fusion protein for use according to any one of the preceding claims, **characterized in that** the fusion protein is embedded in a biodegradable matrix for continuous intraperitoneal release.

13. A pharmaceutical composition comprising a recombinant fusion protein according to any one of claims 1 to 12 for use in the prevention or treatment of peritoneal adhesions following surgical interventions in the peritoneal cavity.

14. The pharmaceutical composition for use according to claim 13, **characterized in that** the recombinant fusion protein is present in an osmotically active medium, preferably Ico-dextrin solution.

## Revendications

1. Protéine de fusion recombinante comprenant une enzyme fibrinogénolytique ayant une séquence d'acides aminés qui est reliée à la terminaison C et/ou à la terminaison N avec la séquence d'acides aminés d'au moins un domaine de stabilisation inerte de poids moléculaire élevé ayant un poids molécule > 50 kDa, destinée à une utilisation lors de la prévention ou du traitement d'adhérences péritonéales après des interventions chirurgicales dans la cavité péritonéale.

2. Protéine de fusion recombinante destinée à une utilisation selon la revendication 1, **caractérisée en ce que** le domaine de stabilisation inerte est relié avec l'enzyme fibrinogénolytique par un agent de liaison.

3. Protéine de fusion recombinante destinée à une utilisation selon la revendication 2, **caractérisée en ce que** l'agent de liaison consiste en un agent de liaison glycine-sérine ayant la séquence (GGGGGS)x ou un agent de liaison glycine-alanine ayant la séquence (GGGGA)xR, avec A = alanine ; G = glycine ; R = arginine ; S = sérine ; x = nombre de répétitions > 1.

4. Protéine de fusion recombinante destinée à une utilisation selon la revendication 1, **caractérisée en ce que** plusieurs domaines de stabilisation inertes sont couplés à la terminaison C de l'enzyme fibrinogénolytique.

5. Protéine de fusion recombinante destinée à une utilisation selon la revendication 1, **caractérisée en ce que** plusieurs domaines de stabilisation inertes sont couplés à la terminaison N de l'enzyme fibrinogénolytique.

6. Protéine de fusion recombinante destinée à une utilisation selon la revendication 1, **caractérisée en ce que** le domaine de stabilisation présente un poids moléculaire compris entre 50 et 150 kDa.

7. Protéine de fusion recombinante destinée à une utilisation selon l'une quelconque des revendications précédentes, **caractérisée en ce que** l'enzyme fibrinogénolytique consiste en une protéase à sérine analogue à la thrombine.

8. Protéine de fusion recombinante destinée à une utilisation selon l'une quelconque des revendications précédentes, **caractérisée en ce que** l'enzyme fibrinogénolytique consiste en l'ancrod ou la batroxobine, ou une variante recombinante d'ancrod ou de batroxobine.

9. Protéine de fusion recombinante destinée à une utilisation selon l'une quelconque des revendications précédentes, **caractérisée en ce que** le domaine de stabilisation inerte de poids moléculaire élevé consiste en un domaine de protéine ou de peptide, une albumine sérique, une transferrine, un anticorps monoclonal ou humanisé ou un fragment d'anticorps, ou un domaine synthétique, p. ex. PAS ou XTEN.

10. Protéine de fusion recombinante destinée à une utilisation selon la revendication 1, **caractérisée en ce que** la séquence d'acides aminés de l'albumine sérique humaine, ou une partie de cette séquence ayant un poids moléculaire > 50 kDa, est couplée en tant que domaine de stabilisation inerte à la terminaison C ou à la terminaison N de l'ancrod ou de la batroxobine en tant qu'enzyme fibrinogénolytique.

11. Protéine de fusion recombinante destinée à une utilisation selon la revendication 10, **caractérisée en ce que** la protéine de fusion comprend une séquence d'acides aminés telle qu'indiquée dans SEQ ID NO. 2 (fusion N-terminale) ou SEQ ID NO. 4 (fusion C-terminale), ou des parties à activité fibrinogénolytique de cette séquence.

12. Protéine de fusion recombinante destinée à une utilisation selon l'une quelconque des revendications précédentes, **caractérisée en ce que** la protéine de fusion se présente sous forme incorporée dans une matrice biodégradable pour la libération intrapéritonéale continue.

13. Composition pharmaceutique comprenant une protéine de fusion recombinante selon l'une quelconque des revendications 1 à 12 destinée à une utilisation lors de la prévention ou du traitement d'adhérences péritonéales après des interventions chirurgicales dans la cavité péritonéale.

14. Composition pharmaceutique destinée à une utilisation selon la revendication 13, **caractérisée en ce que** la composition comprend un milieu osmotiquement actif, la protéine de fusion recombinante se présentant dans le milieu osmotiquement actif, de préférence une solution d'icodextrine.


### SEQUENCE LISTING

<110> Akesion GmbH
<120> Rekombinantes Fusionsproteine zur Vorbeugung oder Behandlung von Adhäsionen bei Geweben oder Organen.
<130> AK01
<150> DE102014112212.7
   <151> 2014-08-26
<160> 4
<170> PatentIn version 3.5
<210> 1
   <211> 1509
   <212> DNA
   <213> Artificial
<220>
   <223> Fusion zwischen Ancrod und des humanen Fc IgG1 Antikörpers
<400> 1
   gcggccgcca ccatgaaatg ggtcaccttt atctcccttc tgttcctctt tagtagcgcc      60
   tattctgtca tcggtggtga cgagtgcaat atcaacgagc atcgatttct ggtggcagtg     120
   tatgaaggaa ccaactggac ctttatctgc ggcggggtcc ttattcaccc agagtgggtc     180
   attaccgccg aacactgtgc tcggcgtcga atgaatcttg tgttcgggat gcacaggaaa     240
   tcagagaagt ttgatgacga acaggaacgg tatcccaaga agcggtactt cattcgatgc     300
   aacaaaaccc ggactagctg ggatgaggac atcatgctga ttcggctgaa caagcccgtg     360
   aataacagcg agcatattgc tcctttgtca ctgccttcca atccgcctat tgtgggtagt     420
   gactgccgtg tgatgggctg gggtagcatt aacagaagga tccacgtgct tagcgatgaa     480
   cccagatgtg ccaacatcaa tctccacaac ttcaccatgt gtcatgggtt gttccgcaag     540
   atgcctaaga agggacgcgt actctgtgct ggcgatctgc gcggtagacg ggactcttgc     600
   aattcagata gtggaggacc ccttatctgc aacgaagagc tgcatggcat tgtggccaga     660
   ggccccaatc catgtgcaca gcccaacaaa ccagctctgt atactagcgt gtacgactac     720
   agggattggg tgaacaacgt tatcgccggc aatgcaacct gtagtccagg cggcggcgga     780
   gccggtggag gcggggcagg aggaggagga gctagagaca aaacacacac ttgtccaccc     840
   tgtcctgctc ccgaactgct tggtggaccc agcgtgtttc tgtttccgcc taagcccaaa     900
   gacaccctca tgatctcacg gactcccgaa gttacgtgtg tcgtagtaga cgtgtcacac     960
   gaagatcccg aggtcaagtt caactggtat gtggacggag ttgaggttca caacgccaaa    1020
   accaaaccga gagaggagca gtacaactcc acatataggg tggtaagcgt gttgaccgtg    1080
   ctgcatcagg attggctgaa tggcaaagag tacaagtgca aggtgtccaa taaggctctt    1140
   ccagcaccca ttgagaaaac gatctccaag gcgaaaggcc aacctcgtga acctcaggtg    1200
   tatactctcc ctccaagtcg cgatgagctc accaagaacc aggtgtcttt gacatgcctc    1260
   gtcaaagggt tctacccatc agacatagcc gtcgaatggg agtctaatgg ccaaccagag    1320
   aataactaca agaccactcc tccggttctg gatagtgatg ggagcttctt tctgtacagc    1380
   aagctgacag tcgacaagtc ccgatggcag cagggtaatg tgttcagttg ctctgtgatg    1440
   catgaagccc tgcataacca ctatacccag aaaagcctgt ctctgagccc aggaaagtaa    1500
   tagaagctt                                                            1509
<210> 2
   <211> 495
   <212> PRT
   <213> Artificial
<220>
   <223> Fusion zwischen Ancrod und des humanen Fc IgG1 Antikörpers
<400> 2
   Met Lys Trp Val Thr Phe Ile Ser Leu Leu Phe Leu Phe Ser Ser Ala
   1               5                   10                  15
   Tyr Ser Val Ile Gly Gly Asp Glu Cys Asn Ile Asn Glu His Arg Phe
               20                  25                  30
   Leu Val Ala Val Tyr Glu Gly Thr Asn Trp Thr Phe Ile Cys Gly Gly
           35                  40                  45
   Val Leu Ile His Pro Glu Trp Val Ile Thr Ala Glu His Cys Ala Arg
       50                  55                  60
   Arg Arg Met Asn Leu Val Phe Gly Met His Arg Lys Ser Glu Lys Phe
   65                  70                  75                  80
   Asp Asp Glu Gln Glu Arg Tyr Pro Lys Lys Arg Tyr Phe Ile Arg Cys
                   85                  90                  95
   Asn Lys Thr Arg Thr Ser Trp Asp Glu Asp Ile Met Leu Ile Arg Leu
               100                 105                 110
   Asn Lys Pro Val Asn Asn Ser Glu His Ile Ala Pro Leu Ser Leu Pro
           115                 120                 125
   Ser Asn Pro Pro Ile Val Gly Ser Asp Cys Arg Val Met Gly Trp Gly
       130                 135                 140
   Ser Ile Asn Arg Arg Ile His Val Leu Ser Asp Glu Pro Arg Cys Ala
   145                 150                 155                 160
   Asn Ile Asn Leu His Asn Phe Thr Met Cys His Gly Leu Phe Arg Lys
                   165                 170                 175
   Met Pro Lys Lys Gly Arg Val Leu Cys Ala Gly Asp Leu Arg Gly Arg
               180                 185                 190
   Arg Asp Ser Cys Asn Ser Asp Ser Gly Gly Pro Leu Ile Cys Asn Glu
           195                 200                 205
   Glu Leu His Gly Ile Val Ala Arg Gly Pro Asn Pro Cys Ala Gln Pro
       210                 215                 220
   Asn Lys Pro Ala Leu Tyr Thr Ser Val Tyr Asp Tyr Arg Asp Trp Val
   225                 230                 235                 240
   Asn Asn Val Ile Ala Gly Asn Ala Thr Cys Ser Pro Gly Gly Gly Gly
                   245                 250                 255
   Ala Gly Gly Gly Gly Ala Gly Gly Gly Gly Ala Arg Asp Lys Thr His
               260                 265                 270
   Thr Cys Pro Pro Cys Pro Ala Pro Glu Leu Leu Gly Gly Pro Ser Val
           275                 280                 285
   Phe Leu Phe Pro Pro Lys Pro Lys Asp Thr Leu Met Ile Ser Arg Thr
       290                 295                 300
   Pro Glu Val Thr Cys Val Val Val Asp Val Ser His Glu Asp Pro Glu
   305                 310                 315                 320
   Val Lys Phe Asn Trp Tyr Val Asp Gly Val Glu Val His Asn Ala Lys
                   325                 330                 335
   Thr Lys Pro Arg Glu Glu Gln Tyr Asn Ser Thr Tyr Arg Val Val Ser
               340                 345                 350
   Val Leu Thr Val Leu His Gln Asp Trp Leu Asn Gly Lys Glu Tyr Lys
           355                 360                 365
   Cys Lys Val Ser Asn Lys Ala Leu Pro Ala Pro Ile Glu Lys Thr Ile
       370                 375                 380
   Ser Lys Ala Lys Gly Gln Pro Arg Glu Pro Gln Val Tyr Thr Leu Pro
   385                 390                 395                 400
   Pro Ser Arg Asp Glu Leu Thr Lys Asn Gln Val Ser Leu Thr Cys Leu
   405 410 415
   Val Lys Gly Phe Tyr Pro Ser Asp Ile Ala Val Glu Trp Glu Ser Asn
   420 425 430
   Gly Gln Pro Glu Asn Asn Tyr Lys Thr Thr Pro Pro Val Leu Asp Ser
   435 440 445
   Asp Gly Ser Phe Phe Leu Tyr Ser Lys Leu Thr Val Asp Lys Ser Arg
   450 455 460
   Trp Gln Gln Gly Asn Val Phe Ser Cys Ser Val Met His Glu Ala Leu
   465 470 475 480
   His Asn His Tyr Thr Gln Lys Ser Leu Ser Leu Ser Pro Gly Lys
   485 490 495
<210> 3
   <211> 2618
   <212> DNA
   <213> Artificial
<220>
   <223> Fusion zwischen Ancrod und humanem Serumalbumin
<400> 3
   gcggccgctc tagagccacc atgaaatggg ttaccttcat tagcctcctg ttcctgtttt      60
   cctccgccta ttctgttatc ggtggtgacg agtgtaacat caacgagcat aggttcctgg     120
   tcgcagtgta tgagggcaca aactggacct tcatttgtgg cggggtgctg attcacccag     180
   agtgggtaat aacagcggag cattgtgccc gcagacgcat gaatctcgtg tttggaatgc     240
   atcgcaaaag cgagaaattc gatgatgaac aagaaaggta ccctaagaag cggtacttca     300
   ttcggtgcaa caagacaaga acttcatggg acgaggacat catgctgatc cgtcttaaca     360
   agccggtaaa taacagcgag catatcgcac cactctcatt gcccagcaac cctcccatcg     420
   tgggaagcga ttgcagagtg atggggtggg gctccatcaa tagaaggatt cacgtgctct     480
   ctgatgaacc gcggtgtgcc aacattaatc tgcataattt tactatgtgc catggtctgt     540
   ttcgcaaaat gcccaagaaa ggaagagttc tgtgtgcagg cgatctgaga ggaaggagag     600
   actcttgcaa ctccgatagt ggcgggccac tgatatgcaa cgaagagctt cacggaatcg     660
   tggccagagg tcctaatcca tgtgctcagc ctaacaagcc cgctctgtac accagcgttt     720
   atgactaccg ggattgggtc aacaatgtca ttgccggaaa tgccacctgt tcccctggcg     780
   gcggcgggtc aggaggagga gggtctggtg gcggcgggtc tgacgcacat aaaagcgaag     840
   tggctcaccg gtttaaagat ctcggcgaag agaacttcaa agctcttgta ttgattgcct     900
   tcgctcagta cttgcaacag tgccctttcg aggaccacgt gaaactggtg aatgaagtca     960
   cagaattcgc taagacgtgt gtggcggatg agagtgctga gaactgtgac aagagtctgc      1020
   acaccctgtt tggggataaa ctgtgcactg tcgctactct gcgagaaact tatggcgaaa      1080
   tggccgactg ctgcgccaag caggaacccg agagaaatga atgctttctg cagcacaaag      1140
   acgacaaccc taatctgcca cgattggttc ggcccgaggt ggacgtaatg tgcacggctt      1200
   tccacgacaa tgaggaaacc ttcctgaaga agtatctcta cgaaatagct cgacggcatc      1260
   cctactttta tgcacccgag ctgctgttct ttgcgaagcg ctataaggcc gctttcacag      1320
   aatgctgtca agctgccgac aaggctgcct gtctcctccc aaaactggac gagctccgcg      1380
   atgaggggaa ggcaagcagt gccaaacagc gcctgaaatg cgcatcactt cagaaattcg      1440
   gagagcgcgc attcaaagca tgggcagtgg ctcgattgtc ccagcgattt cctaaggctg      1500
   aatttgccga agtgtcaaag ctggtgacag accttaccaa agtccacaca gaatgctgcc      1560
   atggtgactt gctggagtgc gccgatgaca gagccgatct ggccaagtac atctgtgaaa      1620
   atcaggattc catctcctcc aaactgaaag aatgctgcga gaaacccctg ctggagaaga      1680
   gccattgtat tgctgaggtg gaaaacgatg agatgccagc ggacctccca tcactggcag      1740
   ccgacttcgt cgagagtaag gacgtgtgta agaactacgc cgaagcgaag gatgtgtttc      1800
   tcgggatgtt tctgtacgaa tatgcgcgtc gtcatcccga ttatagcgtg gttctgctgc      1860
   ttaggcttgc caagacttac gaaaccaccc tcgagaagtg ttgtgccgcc gctgacccgc      1920
   atgagtgcta cgccaaagta tttgacgagt ttaagcctct ggtcgaggag cctcagaacc      1980
   tgatcaaaca gaactgcgag cttttcgagc agttgggtga atacaaattt cagaatgccc      2040
   tgctcgtcag gtatactaag aaggtgcccc aagtgtctac acctaccttg gttgaggtca      2100
   gccggaatct cggcaaggtc ggcagcaaat gctgtaagca cccagaggca aagcgtatgc      2160
   catgtgcaga ggattatctg agtgtcgtcc tcaaccagct gtgcgtactt cacgaaaaga      2220
   caccagtgtc cgatagggtc actaaatgtt gcaccgaatc tctggtgaat cggaggccct      2280
   gtttctcagc tctggaagtt gatgaaacct acgttccgaa ggagttcaat gcagaaacgt      2340
   ttacctttca cgctgacatc tgcacgctct ctgagaagga gaggcagata aagaagcaaa      2400
   cagccctggt agagctggtt aaacacaagc ccaaagcaac aaaggagcag ctgaaagcgg      2460
   tgatggatga cttcgccgcg tttgtggaga agtgctgtaa ggccgacgat aaagaaactt      2520
   gcttcgccga agagggaaag aagcttgtgg cagctagcca agcagccctt gggttgcacc      2580
   accatcacca ccactaatag ccactgtgct ggttcgaa                              2618
<210> 4
   <211> 858
   <212> PRT
   <213> Artificial
<220>
   <223> Fusion zwischen Ancrod und humanem Serumalbumin
<400> 4
   Met Lys Trp Val Thr Phe Ile Ser Leu Leu Phe Leu Phe Ser Ser Ala
   1               5                   10                  15
   Tyr Ser Val Ile Gly Gly Asp Glu Cys Asn Ile Asn Glu His Arg Phe
               20                  25                  30
   Leu Val Ala Val Tyr Glu Gly Thr Asn Trp Thr Phe Ile Cys Gly Gly
           35                  40                  45
   Val Leu Ile His Pro Glu Trp Val Ile Thr Ala Glu His Cys Ala Arg
       50                  55                  60
   Arg Arg Met Asn Leu Val Phe Gly Met His Arg Lys Ser Glu Lys Phe
   65                  70                  75                  80
   Asp Asp Glu Gln Glu Arg Tyr Pro Lys Lys Arg Tyr Phe Ile Arg Cys
                   85                  90                  95
   Asn Lys Thr Arg Thr Ser Trp Asp Glu Asp Ile Met Leu Ile Arg Leu
               100                 105                 110
   Asn Lys Pro Val Asn Asn Ser Glu His Ile Ala Pro Leu Ser Leu Pro
           115                 120                 125
   Ser Asn Pro Pro Ile Val Gly Ser Asp Cys Arg Val Met Gly Trp Gly
       130                 135                 140
   Ser Ile Asn Arg Arg Ile His Val Leu Ser Asp Glu Pro Arg Cys Ala
   145                 150                 155                 160
   Asn Ile Asn Leu His Asn Phe Thr Met Cys His Gly Leu Phe Arg Lys
                   165                 170                 175
   Met Pro Lys Lys Gly Arg Val Leu Cys Ala Gly Asp Leu Arg Gly Arg
               180                 185                 190
   Arg Asp Ser Cys Asn Ser Asp Ser Gly Gly Pro Leu Ile Cys Asn Glu
           195                 200                 205
   Glu Leu His Gly Ile Val Ala Arg Gly Pro Asn Pro Cys Ala Gln Pro
       210                 215                 220
   Asn Lys Pro Ala Leu Tyr Thr Ser Val Tyr Asp Tyr Arg Asp Trp Val
   225                 230                 235                 240
   Asn Asn Val Ile Ala Gly Asn Ala Thr Cys Ser Pro Gly Gly Gly Gly
   245 250 255
   Ser Gly Gly Gly Gly Ser Gly Gly Gly Gly Ser Asp Ala His Lys Ser
   260 265 270
   Glu Val Ala His Arg Phe Lys Asp Leu Gly Glu Glu Asn Phe Lys Ala
   275 280 285
   Leu Val Leu Ile Ala Phe Ala Gln Tyr Leu Gln Gln Cys Pro Phe Glu
   290 295 300
   Asp His Val Lys Leu Val Asn Glu Val Thr Glu Phe Ala Lys Thr Cys
   305 310 315 320
   Val Ala Asp Glu Ser Ala Glu Asn Cys Asp Lys Ser Leu His Thr Leu
   325 330 335
   Phe Gly Asp Lys Leu Cys Thr Val Ala Thr Leu Arg Glu Thr Tyr Gly
   340 345 350
   Glu Met Ala Asp Cys Cys Ala Lys Gln Glu Pro Glu Arg Asn Glu Cys
   355 360 365
   Phe Leu Gln His Lys Asp Asp Asn Pro Asn Leu Pro Arg Leu Val Arg
   370 375 380
   Pro Glu Val Asp Val Met Cys Thr Ala Phe His Asp Asn Glu Glu Thr
   385 390 395 400
   Phe Leu Lys Lys Tyr Leu Tyr Glu Ile Ala Arg Arg His Pro Tyr Phe
   405 410 415
   Tyr Ala Pro Glu Leu Leu Phe Phe Ala Lys Arg Tyr Lys Ala Ala Phe
   420 425 430
   Thr Glu Cys Cys Gln Ala Ala Asp Lys Ala Ala Cys Leu Leu Pro Lys
   435 440 445
   Leu Asp Glu Leu Arg Asp Glu Gly Lys Ala Ser Ser Ala Lys Gln Arg
   450 455 460
   Leu Lys Cys Ala Ser Leu Gln Lys Phe Gly Glu Arg Ala Phe Lys Ala
   465 470 475 480
   Trp Ala Val Ala Arg Leu Ser Gln Arg Phe Pro Lys Ala Glu Phe Ala
   485 490 495
   Glu Val Ser Lys Leu Val Thr Asp Leu Thr Lys Val His Thr Glu Cys
   500 505 510
   Cys His Gly Asp Leu Leu Glu Cys Ala Asp Asp Arg Ala Asp Leu Ala
   515 520 525
   Lys Tyr Ile Cys Glu Asn Gln Asp Ser Ile Ser Ser Lys Leu Lys Glu
   530 535 540
   Cys Cys Glu Lys Pro Leu Leu Glu Lys Ser His Cys Ile Ala Glu Val
   545 550 555 560
   Glu Asn Asp Glu Met Pro Ala Asp Leu Pro Ser Leu Ala Ala Asp Phe
   565 570 575
   Val Glu Ser Lys Asp Val Cys Lys Asn Tyr Ala Glu Ala Lys Asp Val
   580 585 590
   Phe Leu Gly Met Phe Leu Tyr Glu Tyr Ala Arg Arg His Pro Asp Tyr
   595 600 605
   Ser Val Val Leu Leu Leu Arg Leu Ala Lys Thr Tyr Glu Thr Thr Leu
   610 615 620
   Glu Lys Cys Cys Ala Ala Ala Asp Pro His Glu Cys Tyr Ala Lys Val
   625 630 635 640
   Phe Asp Glu Phe Lys Pro Leu Val Glu Glu Pro Gln Asn Leu Ile Lys
   645 650 655
   Gln Asn Cys Glu Leu Phe Glu Gln Leu Gly Glu Tyr Lys Phe Gln Asn
   660 665 670
   Ala Leu Leu Val Arg Tyr Thr Lys Lys Val Pro Gln Val Ser Thr Pro
   675 680 685
   Thr Leu Val Glu Val Ser Arg Asn Leu Gly Lys Val Gly Ser Lys Cys
   690 695 700
   Cys Lys His Pro Glu Ala Lys Arg Met Pro Cys Ala Glu Asp Tyr Leu
   705 710 715 720
   Ser Val Val Leu Asn Gln Leu Cys Val Leu His Glu Lys Thr Pro Val
   725 730 735
   Ser Asp Arg Val Thr Lys Cys Cys Thr Glu Ser Leu Val Asn Arg Arg
               740                 745                 750
   Pro Cys Phe Ser Ala Leu Glu Val Asp Glu Thr Tyr Val Pro Lys Glu
           755                 760                 765
   Phe Asn Ala Glu Thr Phe Thr Phe His Ala Asp Ile Cys Thr Leu Ser
       770                 775                 780
   Glu Lys Glu Arg Gln Ile Lys Lys Gln Thr Ala Leu Val Glu Leu Val
   785                 790                 795                 800
   Lys His Lys Pro Lys Ala Thr Lys Glu Gln Leu Lys Ala Val Met Asp
                   805                 810                 815
   Asp Phe Ala Ala Phe Val Glu Lys Cys Cys Lys Ala Asp Asp Lys Glu
               820                 825                 830
   Thr Cys Phe Ala Glu Glu Gly Lys Lys Leu Val Ala Ala Ser Gln Ala
           835                 840                 845
   Ala Leu Gly Leu His His His His His His
       850                 855